(19) **Europäisches Patentamt / European Patent Office / Office européen des brevets**

(11) **EP 4 549 420 A1**

(12) **EUROPEAN PATENT APPLICATION**
published in accordance with Art. 153(4) EPC

(43) Date of publication:
07.05.2025 Bulletin 2025/19

(21) Application number: 23830164.2

(22) Date of filing: 26.06.2023

(51) International Patent Classification (IPC):
C07C 5/29 (2006.01)      C07C 13/615 (2006.01)
B01J 29/12 (2006.01)      B01J 29/14 (2006.01)

(52) Cooperative Patent Classification (CPC):
B01J 29/12; B01J 29/14; C07C 5/29; C07C 13/615

(86) International application number:
PCT/CN2023/102310

(87) International publication number:
WO 2024/001986 (04.01.2024 Gazette 2024/01)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 29.06.2022 CN 202210755708
23.05.2023 CN 202310579206

(71) Applicants:
• China Petroleum & Chemical Corporation
  Beijing 100728 (CN)
• Sinopec Research Institute of Petroleum
  Processing Co., Ltd.
  Beijing 100083 (CN)

(72) Inventors:
• FU, Zhaolin
  Beijing 100083 (CN)
• ZHAO, Jie
  Beijing 100083 (CN)
• TAO, Zhiping
  Beijing 100083 (CN)
• YAN, Rui
  Beijing 100083 (CN)
• JIA, Dandan
  Beijing 100083 (CN)
• ZHU, Zhongpeng
  Beijing 100083 (CN)

(74) Representative: Hoffmann Eitle
Patent- und Rechtsanwälte PartmbB
Arabellastraße 30
81925 München (DE)

(54) **METHOD FOR PREPARING ADAMANTANE**

(57) The application discloses a continuous process for producing adamantane, comprising the steps of: 1) providing a liquid feed stream comprising endo-tetrahydrodicyclopentadiene; 2) passing the liquid feed stream through a first reaction zone filled with a hydrogenation protective agent and a second reaction zone filled with an isomerization catalyst sequentially, to carry out hydroisomerization reaction, thereby obtaining the adamantane. The process has simple process operation and no pollution, can realize long-period continuous preparation of the adamantane, and has practical industrial application prospect.

EP 4 549 420 A1

**Description**

Technical Field

The application relates to the field of hydrocarbon preparation, in particular to a process for producing adamantane.

Background

Adamantane (ADH) is a highly symmetric polycyclic cage hydrocarbon compound, has the molecular formula of $C_{10}C_{16}$, and has the characteristics of high density, good thermal stability, fat solubility, and the like. Hydrogen atoms on its ring can perform substitution reactions and oxidation reactions, such as halogenation reactions, nitration reactions, sulfonation reactions, and the like. It has very wide application in the fields of synthesis of drug intermediates, development of novel materials, preparation of lubricating oil and high-density liquid fuel, and the like.

At present, methods for synthesizing adamantane mainly include an aluminum trichloride method, a molecular sieve method, a super-strong acid method, an ionic liquid method, and the like. In the methods, adamantane is mainly prepared by taking endo-tetrahydrodicyclopentadiene (endo-THDCPD) as an initial raw material to perform an isomerization reaction. The main products of substances involved in the reaction process are adamantane and the isomers thereof as exo-tetrahydrodicyclopentadiene (exo-THDCPD). The specific reaction process is shown in the following figure.

exo-THDCPD

endo-THDCPD

ADH

The reaction mechanism for preparing adamantane is an acid-catalyzed carbonium ion reaction. Acid is required as a catalyst. Olefin intermediates can be generated in the reaction process. The olefin intermediates are easy to generate excessive tar by-products under the strong acid catalysis effect, to form a competitive reaction with the generation of adamantine. This results in the low selectivity of adamantane and serious material loss. However, when the acid is not strong enough or the reaction conditions are too mild, the formation of adamantane cannot be promoted, so the product mainly comprises a slightly isomeric product, namely exo-tetrahydrodicyclopentadiene. The selectivity of adamantane is also low.

Among the methods, the aluminum trichloride method has the characteristics of high conversion rate and high selectivity. The conversion rate can reach 95% or more, the selectivity can reach 50%, and the residue is mainly tar products. The aluminum trichloride method is currently used to produce adamantane industrially. But this method is a method for batch-producing adamantane by taking a reaction kettle as a reactor, and the problems of high toxicity, complex post-treatment, incapability of recycling a catalyst, and high tar generation amount exist in the method. Therefore, the technique for producing adamantane has low total output, high cost, and serious pollution, and cannot meet the development trend of green and low-carbon chemical industry in the future. The article "Synthesis of adamantane on commercially available zeolitic catalysts, Applied Catalysis A: General, 2000, 127-132." studies the preparation of adamantane by isomerization of endo-THDCPD catalyzed by different molecular sieves. When Hβ is used as the catalyst, the yield of adamantane is 15.9%, but a large amount of tar is generated in the process, with a yield of 60%, which does not have the potential for industrial application. The article "Synthesis of adamantane on PW/USY composite catalyst, high-grade chemical engineering report, 2007, 127-132" discloses that PW/USY catalyst loaded with 10% phosphotungstic acid is used for isomerization of endo-THDCPD into adamantane, and a yield of 28.3% is obtained. But this reaction is carried out in a reaction kettle. Carbon deposits are formed on the surface of the PW/USY catalyst after each reaction. High-temperature calcination regeneration is required. The regeneration is frequent, and continuous production capacity is not provided. The patent CN 1398245A discloses a catalyst for producing adamantane, wherein the catalyst is prepared with a Y-type molecular sieve by an ion exchange process to load group VIII metals. A continuous reaction occurs in a stainless steel reaction tube. When the conversion rate of propylene norbornane (i.e., endo-THDCPD) is up to 95.2% at 350 °C, the selectivity of adamantane is 12.8%. There are further improvement spaces for the conversion rate and the selectivity.

The continuous production of adamantane is not realized in the literature reported in the prior art, and particularly the problem that the catalyst is easy to coke and deactivate is not solved, so there is still a need to develop a proper catalyst and a corresponding process.

Disclosure of Invention

The purpose of the present application is to provide a process for producing adamantane, which enables the production of adamantane from endo-tetrahydrodicyclopentadiene as a raw material with a high conversion and a high selectivity and is suitable for a continuous long-period stable production of adamantane.

To achieve the above purpose, in one aspect, the present application provides a continuous process for producing adamantane, comprising the steps:

1) providing a liquid feed stream comprising endo-tetrahydrodicyclopentadiene;
2) passing the liquid feed stream through a first reaction zone filled with a hydrogenation protective agent and a second reaction zone filled with an isomerization catalyst sequentially, to carry out hydroisomerization reaction, thereby obtaining adamantane,
wherein the reaction temperature in the first reaction zone is from 120 to 300 °C, the reaction temperature in the second reaction zone is from 181 to 300 °C, the hydrogenation protective agent is a loaded metal hydrogenation catalyst, and the isomerization catalyst is a metal modified molecular sieve catalyst.

Preferably, step 1) further comprises pretreating the liquid feed stream comprising endo-tetrahydrodicyclopentadiene with an adsorbent.

In another aspect, the present application provides a process for producing adamantane, comprising contacting a liquid feed stream containing endo-tetrahydrodicyclopentadiene with an isomerization catalyst under a hydrogen atmosphere to perform an isomerization reaction, wherein the isomerization catalyst is a metal-modified molecular sieve catalyst, with a specific surface area of from 450 to 900 $m^2$/g, a pore volume of from 0.25 to 0.5 $cm^3$/g, a mesoporous volume of from 0.02 to 0.10 $cm^3$/g, a strong acid amount of from 150 to 850 $\mu$mol/g, a reaction temperature of from 181 to 300 °C, and a reaction pressure of from 0.1 to 3.0MPa.

The application, on one hand, allows the endo-tetrahydrodicyclopentadiene reaction raw material to pass through a first reaction zone filled with a hydrogenation protective agent and a second reaction zone filled with an isomerization catalyst sequentially to carry out a hydroisomerization reaction, preferably after an adsorption pretreatment. This can greatly improve the service life of the isomerization catalyst and the process stability, accomplishing green and continuous stable production of adamantane. The conversion rate can be 99%, and the selectivity of the target product adamantane can be 15.9%, without tar generated. Its atom utilization rate is high. The process can accomplish a continuous operation for more than 500 hours and has an industrial application prospect.

In addition, on the other hand, in the application, the hydroisomerization reaction of the endo-tetrahydrodicyclopentadiene is carried out in the presence of the metal/molecular sieve-supported bifunctional catalyst with specific properties, so that the activity and stability of the catalyst in the reaction process can be improved, the coking and inactivation of the catalyst can be obviously inhibited, the reaction conversion rate and selectivity are further improved, and the process is suitable for a continuous long-period stable production of adamantane.

Additional features and advantages of the application will be set forth in the following detailed description.

Detailed Description

The embodiments of the present application are described in detail below. It should be understood that the embodiments here are only employed for indicating and explaining the application, without any limitation for the application.

Any specific value disclosed herein (including endpoints of ranges for values) is not limited to the precise value of that value and is to be understood to also encompass values close to the precise value, for example, all possible values ±5% of the precise value. Also, for the disclosed ranges of values, any combination between the endpoints of the range, between the endpoints of the range and the specific points within the range, and between the specific points can result in one or more new ranges of values, and such new ranges of values should also be regarded as being specifically disclosed herein.

Unless otherwise defined, terms used herein have the same meaning as commonly understood by one of ordinary skill in the art, and if a term is defined herein and its definition is different from the definition commonly understood in the art, the definition herein controls.

In the present application, anything or things that are not mentioned are directly applicable to those known in the art without any change except what is explicitly stated. Moreover, any embodiment described in this document may be freely combined with one or more other embodiments described in this document, and the technical solutions or technical ideas formed thereby are deemed to be part of the original disclosure or original record of this application, and should not be regarded as new content that has not been disclosed or anticipated in this document, unless those skilled in the art considers that the combination is obviously unreasonable.

In the present application, unless otherwise specified, the pressure values are given as gauge pressures.

In the present application, the metal contents of the hydrogenation protective agent and the isomerization catalyst are given as metal elements unless otherwise specified.

In the present application, the term "the volume ratio of hydrogen to liquid" refers to the volume ratio of hydrogen gas to liquid material in the reaction system, in units of $Nm^3/m^3$.

In the present application, the specific surface area and pore volume (including mesoporous volume) of a catalyst are measured using a nitrogen isothermal physical adsorption-desorption process (BET).

In the present application, the total acid amount and the strong acid amount of the catalyst are measured by pyridine absorption infrared spectroscopy process (Py-IR).

All patent and non-patent documents referred to herein, including but not limited to textbooks and journal articles and the like, are incorporated by reference in their entirety.

As described above, in a first aspect, the present application provides a continuous process for producing adamantane, comprising the steps:

1) providing a liquid feed stream comprising endo-tetrahydrodicyclopentadiene;
2) passing the liquid feed stream through a first reaction zone filled with a hydrogenation protective agent and a second reaction zone filled with an isomerization catalyst sequentially, to carry out a hydroisomerization reaction, thereby obtaining adamantane,
wherein the reaction temperature in the first reaction zone is from 120 to 300 °C, preferably from 151 to 250 °C, more preferably from 171 to 220 °C; the reaction temperature in the second reaction zone is from 181 to 300 °C, preferably from 200 to 260 °C, the hydrogenation protective agent is a metal loaded hydrogenation catalyst, and the isomerization catalyst is a metal modified molecular sieve catalyst.

According to the present application, there is no particular requirement for endo-tetrahydrodicyclopentadiene as a starting material, which can be prepared in the various ways disclosed in the prior art or is commercially available.

In a preferred embodiment, the liquid feed stream comprises endo-tetrahydrodicyclopentadiene and a reaction solvent selected from hydrocarbon or halogenated hydrocarbon solvents having a boiling point of from 40 to 300 °C, such as cyclohexane, methylcyclohexane, dichloromethane, exo-tetrahydrodicyclopentadiene and the like, wherein the hydrocarbon or halogenated hydrocarbon solvents are preferably selected from C6-C10 hydrocarbons, more preferably from cyclohexane, methylcyclohexane, exo-tetrahydrodicyclopentadiene, or combinations thereof. Preferably, the concentration by mass of the endo-tetrahydrodicyclopentadiene in the feed stream is from 10 to 80%, more preferably from 30 to 60%.

In a preferred embodiment, said step 1) further comprises pretreating said liquid feed stream comprising endo-tetrahydrodicyclopentadiene with an adsorbent. Without being limited to a particular theory, the inventors of the present application found that the removal of water, sulfides, nitrides, and oxygen-containing compounds, etc. from the liquid feed stream by adsorption pretreatment can significantly improve the stability of the reaction system, facilitating continuous long-term stable production of adamantane.

In a further preferred embodiment, the adsorbent is selected from activated clay, NaY molecular sieve, X-type molecular sieve, activated carbon, or combinations thereof, more preferably selected from activated clay, NaY molecular sieve, or combinations thereof.

In a still further preferred embodiment, the conditions of said pretreatment of step 1) comprise: the temperature is from normal temperature to 60 °C, the pressure is from 0.0 to 0.5 MPa, and the mass space velocity of the endo-tetrahydrodicyclopentadiene is from 0.1 to 10.0 $h^{-1}$, preferably from 0.2 to 1 $h^{-1}$.

In certain embodiments, the pretreatment of step 1) is accomplished by contacting the adsorbent with a reaction solvent in a pretreatment reactor that is not strictly limited, such as, but not limited to, a fixed bed reactor or a glass reaction tube, and the like.

Without being limited to a specific theory, the inventors of the present application also found that by using the hydrogenation protective agent in step 2), impurities such as trace olefins in the raw material endo-tetrahydrodicyclopentadiene can be removed by hydrogenation and hydrogen can be activated, to protect the isomerization catalyst, inhibit coking thereof and prolong the service life thereof.

In a preferred embodiment, the hydrogenation protective agent comprises a support and an active metal supported on the support, wherein the active metal is selected from the group consisting of noble metals Pd, Pt, Ru, Rh, non-noble metals Ni, or combinations thereof, preferably Ni, Pd, Pt, or combinations thereof; the support is selected from non-acidic supports, such as $Al_2O_3$, $SiO_2$, $ZrO_2$, $TiO_2$, $CeO_2$, activated carbon, or combinations thereof, preferably $Al_2O_3$, $SiO_2$, or combinations thereof. In a further preferred embodiment, based on the total mass of the hydrogenation protect agent, the non-noble metal (Ni) content of the hydrogenation protect agent is from 1 to 40%, preferably from 5 to 30%, more preferably from 10 to 20%; and/or the total content of noble metals (Pd, Pt, Ru, and Rh) is from 0.1 to 10%, preferably from 0.2 to 5%, more preferably from 0.3 to 3%.

According to the present application, the hydrogenation protective agent can be prepared by a conventional process or commercially available and is not strictly limited.

In the application, the isomerization reaction in step 2) is carried out under the catalysis of a metal-modified molecular sieve catalyst (i.e. a metal/molecular sieve-supported catalyst), and the catalyst has the double functions of isomerization-coking inhibition. The molecular sieve performs stable isomerization activity by loading metal on the molecular sieve. The metal does not participate in the isomerization reaction, however, the metal inhibits the generation of coking precursor (such as olefin intermediate) in the reaction process, thereby improving the service life of the catalyst.

In a preferred embodiment, the isomerization catalyst comprises a molecular sieve and a modifying metal supported on the molecular sieve, wherein the modifying metal is selected from the noble metals Pd, Pt, Ru, Rh, Au, non-noble metals Ni, or combinations thereof, preferably Pt, Pd, or combinations thereof; the molecular sieve is a Y-type molecular sieve, preferably selected from HY, USY and REHY or combinations thereof, and more preferably selected from HY, HUSY and REHY or combinations thereof. Further preferably, based on the total mass of the isomerization catalyst, the content of non-noble metal (Ni) in the isomerization catalyst is from 1 to 20%, preferably from 3 to 15%, more preferably from 3 to 10%; and/or the total content of noble metals (Pd, Pt, Au, Ru and Rh) is from 0.05 to 5.0%, preferably from 0.1 to 1.0%, more preferably from 0.2 to 0.5%. Even more preferably, the molecular sieve has a $Na_2O$ content of less than 0.5%, preferably less than 0.2%, based on the mass of the molecular sieve.

According to the present application, the isomerization catalyst is commercially available or can be prepared by a conventional process, for example, by an equivalent volume impregnation process, an excess volume impregnation process, or the like. For example, the isomerization catalyst may be prepared by: preparing a certain amount of metal precursor solution according to metal loading amount, then impregnating a molecular sieve with the solution, standing it for more than 6 hours at normal temperature, with a stirring intermittently in the process, then drying it for more than 12 hours at a temperature of from 80 to 120 °C, and calcining it for from 2 to 5 hours at a temperature of from 450 to 550 °C in an air atmosphere. Then, the calcined catalyst is reduced in a reducing atmosphere such as hydrogen at from 400 to 550 °C for from 2 to 5 h to obtain the activated catalyst.

In a preferred embodiment, the isomerization catalyst has a specific surface area of from 450 to 900 $m^2/g$, preferably from 600 to 800 $m^2/g$, a pore volume of from 0.25 to 0.5 $cm^3/g$, preferably from 0.35 to 0.45 $cm^3/g$, a mesoporous volume of from 0.02-0.10 $cm^3/g$, preferably from 0.07 to 0.09 $cm^3/g$, a strong acid amount of from 150 to 850 $\mu$mol/g, preferably from 370 to 700 $\mu$mol/g.

In a further preferred embodiment, the isomerization catalyst is prepared by a process comprising the steps:

I) modifying the Y-type molecular sieve by using an ammonium fluosilicate solution, wherein the mass ratio of the ammonium fluosilicate to the Y-type molecular sieve is from 0.1 to 0.3, and the Y-type molecular sieve is preferably a hydrogen-type molecular sieve, more preferably selected from HY, HUSY, REHY or combinations thereof; and
II) loading the modifying metal on the modified molecular sieve obtained in step I), calcining, and reducing in a reducing atmosphere, to obtain the isomerization catalyst.

In a still further preferred embodiment, the conditions of the modification treatment in step I) include: a treatment temperature of from 30 to 100 °C, preferably from 50 to 80 °C, and a treatment time of from 0.5 to 5 hr, preferably from 0.5 to 3 hr. More preferably, a concentration of the ammonium fluosilicate solution is from 0.02 to 2 mol/L, preferably from 0.05 to 0.3 mol/L.

In a still further preferred embodiment, the loading of step II) is achieved by impregnating the modified molecular sieve with a solution of a precursor of the modifying metal, and optionally drying. Preferably the precursor is a salt of the modifying metal.

In a preferred embodiment, the first and second reaction zones in step 2) are under a hydrogen atmosphere and a reaction pressure is from 0.1 to 3MPa, preferably from 0.5 to 1.0 MPa. More preferably, the mass space velocity of the endo-tetrahydrodicyclopentadiene in the first and second reaction zones is from 0.5 to 5$h^{-1}$, preferably from 0.5 to 2$h^{-1}$; the volume ratio of hydrogen to liquid is from 100 to 1600, preferably from 600 to 1200.

In some specific embodiments, the exo-tetrahydrodicyclopentadiene and the reaction solvent are separated as intermediate products from the materials after the reaction in step 2), thereby an adamantane concentrated solution can be obtained. The concentrated solution is cooled and crystallized to obtain an adamantane crude product, and the adamantane product with higher purity can be obtained through recrystallization. The exo-tetrahydrodicyclopentadiene and the reaction solvent can be recycled. The atom utilization rate in the whole process is high.

In a preferred embodiment, said step 2) is performed using a fixed bed reactor, and the first reaction zone is disposed above the second reaction zone. Preferably the first reaction zone and the second reaction zone are separated by an inert material.

In a more preferred embodiment, the inert material can be selected from $SiO_2$, $Al_2O_3$, carbon materials, quartz sand, and the like, preferably quartz sand.

In certain preferred embodiments, the process of the present application comprises: 1) introducing endo-tetrahydrodicyclopentadiene and a reaction solvent into a pretreatment reactor filled with an adsorbent for pretreatment, and 2) introducing the effluent liquid obtained in step 1) into a fixed bed reactor for hydroisomerization reaction to obtain adamantane; wherein in the fixed bed reactor, the first reaction zone of the upper section is filled with the hydrogenation protective agent, and the second reaction zone of the lower section is filled with the isomerization catalyst.

In certain particularly preferred embodiments, the process of the present application comprises: 1) uniformly mixing endo-tetrahydrodicyclopentadiene with a reaction solvent, introducing it into a pretreatment reactor, and adsorbing and removing impurities with adsorbents such as activated clay, NaY and other; and 2) flowing the effluent liquid in the step 1) into the upper end of the fixed bed reactor, and flowing it out from the lower end of the fixed bed reactor, wherein in the fixed bed reactor, the hydrogenation protective agent is filled in the first reaction zone of the upper section, the isomerization catalyst is filled in the second reaction zone of the lower section, an inert material is arranged between them for separation, wherein the hydrogenation reaction temperature of the upper section is from 120 to 300 °C; the isomerization reaction temperature of the lower section is from 181 to 300 °C, the reaction pressure of the entire fixed bed is from 0.1 to 3MPa hydrogen, the mass space velocity of the endo- tetrahydrodicyclopentadiene is from 0.5 to 5 h$^{-1}$, and the volume ratio of hydrogen to liquid is from 100 to 1600.

According to the application, in some embodiments, the endo-tetrahydrodicyclopentadiene and the reaction solvent are uniformly premixed in a raw material tank, and then flow into a pretreatment reactor filled with an adsorbent from the upper end under normal temperature and normal pressure. The material without impurity flows out from the lower end, then is pumped to the upper end of a fixed bed reactor, passes through a hydrogenation protective agent, an inert material, and an isomerization catalyst reaction bed layer together with hydrogen. The reaction product flows out from the lower end of the fixed bed. The product contains exo-tetrahydrodicyclopentadiene, adamantane, and other ring-opening by-products. Adamantane can be obtained after separation.

According to the application, gas chromatography analysis is performed on the sample taken in step 2). The reactant conversion rate and the product selectivity are calculated according to an area normalization method.

According to the process of the first aspect of the application, endo-tetrahydrodicyclopentadiene is used as a reaction raw material and passes through a first reaction zone filled with a hydrogenation protective agent and a second reaction zone filled with an isomerization catalyst (for example, a fixed bed reactor with an upper section filled with the hydrogenation protective agent and a lower section filled with the isomerization catalyst) sequentially, to carry out hydroisomerization reaction. Preferably, after adsorption pretreatment, the service life and the process stability of the isomerization catalyst can be greatly improved. A green, continuous and stable production of adamantane is realized. The conversion rate can reach 99%, the selectivity of the target product adamantane can reach 15.9%. No tar is generated, and the atom utilization rate is high. A continuous operation for more than 500 hours can be realized, and the process has an industrial application prospect.

In a second aspect, the present application provides an isomerization catalyst for producing adamantane by isomerization of tetrahydrodicyclopentadiene, which comprises a Y-type molecular sieve and a modifying metal supported on the molecular sieve, wherein the catalyst is prepared by a process comprising the steps of:

I) Modifying the Y-type molecular sieve by using an ammonium fluosilicate solution, wherein the mass ratio of the ammonium fluosilicate to the Y-type molecular sieve is from 0.1 to 0.3; and

II) loading the modifying metal on the modified molecular sieve obtained in step I), calcining, and reducing in a reducing atmosphere to obtain the isomerization catalyst.

In a preferred embodiment, the Y-type molecular sieve is a hydrogen-type molecular sieve, more preferably selected from HY, HUSY, REHY, or combinations thereof.

In a preferred embodiment, the Y-type molecular sieve has a $Na_2O$ content of less than 0.5%, preferably less than 0.2%, based on the mass of the molecular sieve.

In a preferred embodiment, the modifying metal is selected from Pt, Pd, Au, Ru, Rh, Ni, or combinations thereof. Further preferably, the modifying metal loading amount is from 0.05 to 5.0%, preferably from 0.2 to 0.5%, based on the total mass of the catalyst, when the modifying metal is Pt, Pd, Au, Ru and/or Rh, and from 1.0 to 10%, preferably from 3 to 6%, when the modifying metal is Ni.

In a preferred embodiment, the modification treatment of step I) is carried out by: mixing the Y-type molecular sieve with water, uniformly stirring it to form slurry, adding an ammonium fluosilicate solution into the slurry of the Y-type molecular sieve according to the requirement that the mass ratio of ammonium fluosilicate to the molecular sieve is from 0.1 to 0.3, treating it from 0.5 to 5 hours, preferably from 0.5 to 3 hours at the temperature of from 30 to 100 °C, preferably from 50 to 80 °C, and then filtering and drying. Further preferably, the mass ratio of the Y-type molecular sieve to water is from 1:1 to 1: 30, preferably from 1:5 to 1:15, thereby forming a slurry.

In a preferred embodiment, the concentration of the ammonium fluorosilicate solution used in step I) is from 0.02 to 2mol/L, preferably from 0.05 to 0.3 mol/L.

In a preferred embodiment, said loading of step II) is achieved by impregnating said modified molecular sieve with a solution of a precursor of said modifying metal, and optionally drying. Preferably the precursor of said modifying metal is a salt of said modifying metal.

In a preferred embodiment, in step II), the calcination comprises calcinating at 350-600 °C, preferably 400-500 °C for 1-6h, preferably 2-4 h; and/or the reduction comprises reducing in a hydrogen atmosphere at from 150 to 600 °C, preferably from 250 to 500 °C for more than 1h, for example, 2-5 h.

According to the isomerization catalyst of the second aspect of the application, the molecular sieve is firstly treated by ammonium fluosilicate solution, so that the effects of expanding pores of the catalyst and enhancing acid content and acid strength can be achieved. The activity and stability of the catalyst are improved. The bifunctional catalyst is prepared by further carrying out metal impregnation on the molecular sieve. The bifunctional catalyst has the characteristic remarkable effect of inhibiting the coking and inactivation of the catalyst and can be used for producing adamantane by isomerization of tetrahydrodicyclopentadiene.

In a third aspect, the present application provides a process for producing adamantane, which comprises contacting a liquid feed stream comprising tetrahydrodicyclopentadiene, particularly endo-tetrahydrodicyclopentadiene, with an isomerization catalyst under a hydrogen atmosphere to effect an isomerization reaction, wherein the isomerization catalyst is a metal modified molecular sieve catalyst, with a specific surface area of from 450 to 900 $m^2/g$, preferably from 600 to 800 $m^2/g$, a pore volume of from 0.25 to 0.5 $cm^3/g$, preferably from 0.35 to 0.45 $cm^3/g$, a mesoporous volume of from 0.02 to 0.10 $cm^3/g$, preferably from 0.07 to 0.09 $cm^3/g$, a strong acid amount of from 150 to 850 $\mu$mol/g, preferably from 370 to 700 $\mu$mol/g, a reaction temperature of from 181 to 300 °C, preferably from 200 to 260 °C; a reaction pressure of from 0.1 to 3.0 MPa, preferably from 0.5 to 1.0 MPa.

In a preferred embodiment, the mass space velocity of the endo-tetrahydrodicyclopentadiene in the isomerization reaction is from 0.2 to $5h^{-1}$, preferably from 0.5 to $2h^{-1}$; the volume ratio of hydrogen to liquid is from 100 to 3000, preferably from 600 to 1200.

In a preferred embodiment, the isomerization catalyst comprises a molecular sieve and a modifying metal loaded on the molecular sieve, wherein the modifying metal is selected from Pd, Pt, Au, Ru, Rh, Ni, or combinations thereof. The molecular sieve is a Y-type molecular sieve, preferably selected from HY, USY, REHY, or combinations thereof, more preferably selected from HY, HUSY, REHY, or combinations thereof. Further preferably, based on the total mass of the isomerization catalyst, the content of Ni in the isomerization catalyst is from 1 to 20%, preferably from 3 to 15%, more preferably from 3 to 10%; and/or the total content of Pd, Pt, Au, Ru, and Rh is from 0.05 to 5.0%, preferably from 0.1 to 1.0%, more preferably from 0.2 to 0.5%. Even more preferably, the molecular sieve has a $Na_2O$ content of less than 0.5%, preferably less than 0.2%, based on the mass of the molecular sieve.

In a particularly preferred embodiment, the isomerization catalyst is prepared by a process comprising the steps of:

I) modifying the Y-type molecular sieve by using an ammonium fluosilicate solution, wherein the mass ratio of the ammonium fluosilicate to the Y-type molecular sieve is from 0.1 to 0.3. The Y-type molecular sieve is preferably a hydrogen-type molecular sieve, more preferably selected from HY, HUSY, REHY, or combinations thereof; and
II) loading modifying metal on the modified molecular sieve obtained in step I), calcining, and reducing in a reducing atmosphere to obtain the isomerization catalyst.

In a further preferred embodiment, the conditions of the modification treatment in step I) include: a treatment temperature of 30-100 °C, preferably 50-80 °C, and a treatment time of 0.5-5 hr, preferably 0.5-3 hr. More preferably, the concentration of the ammonium fluosilicate solution is 0.02-2 mol/L, preferably 0.05-0.3 mol/L.

In a further preferred embodiment, said loading of step II) is achieved by impregnating the modified molecular sieve with a solution of a precursor of said modifying metal, and optionally drying. Preferably the precursor is a salt of said modifying metal.

In a preferred embodiment, the feed stream comprises endo-tetrahydrodicyclopentadiene and a reaction solvent selected from hydrocarbon or halogenated hydrocarbon solvents having a boiling point of from 40 to 300 °C. The hydrocarbon or halogenated hydrocarbon solvents are preferably selected from C6 to C10 hydrocarbons, more preferably from cyclohexane, methylcyclohexane, exo-tetrahydrodicyclopentadiene, or combinations thereof. More preferably, the concentration by mass of the endo-tetrahydrodicyclopentadiene in the feed stream is from 10 to 80%, more preferably from 30 to 60%.

In a preferred embodiment, the isomerization reaction is carried out in a reactor selected from a fixed bed, a moving bed, a fluidized bed, a slurry bed, or combinations thereof, preferably in a fixed bed reactor.

In a preferred embodiment, the process further comprises pretreating the liquid feed stream comprising tetrahydrodicyclopentadiene with an adsorbent prior to the isomerization reaction.

In a further preferred embodiment, the adsorbent is selected from activated clay, NaY molecular sieve, X-type molecular sieve, activated carbon, or combinations thereof, more preferably selected from activated clay, NaY molecular sieve, or combinations thereof.

In a still further preferred embodiment, the conditions of the pretreatment comprise: the temperature from normal temperature to 60 °C, the pressure from 0.0 to 0.5 MPa, and the mass space velocity of the endo-tetrahydrodicyclopentadiene from 0.1 to 10.0 $h^{-1}$, preferably from 0.2 to 1 $h^{-1}$. Other features of the pre-processing step are as described in the first aspect of the present application and will not be described herein again.

In certain preferred embodiments, the present application provides the following:

A1. A continuous process for continuously and stably producing adamantane, comprising: introducing endo-tetrahydrodicyclopentadiene and a reaction solvent into a pretreatment reactor filled with an adsorbent for pretreatment, and introducing an effluent liquid into a fixed bed reactor for hydroisomerization reaction to obtain adamantane; wherein, in the fixed bed reactor, the upper section is filled with a hydrogenation protective agent, the lower section is filled with an isomerization catalyst, the temperature of the upper section hydrogenation reaction is from 120 to 300 °C, and the temperature of the lower section isomerization reaction is from 181 to 300 °C.

A2. The process according to clause A1, wherein the upper section hydrogenation reaction temperature is from 151 to 250 °C; the lower section isomerization reaction temperature is from 200 to 260 °C.

A3. The process according to clause A1, wherein the reaction solvent is selected from solvents with a boiling point of from 40 to 300 °C, preferably C6-C10 hydrocarbons, such as cyclohexane, methylcyclohexane, exo-tetrahydrodicyclopentadiene.

A4. The process according to clause A1, wherein the endo-tetrahydrodicyclopentadiene has a concentration by mass of from 10 to 80%, preferably from 30 to 60%, after mixing with the reaction solvent.

A5. The process according to clause A1, wherein the pretreatment reactor includes, but is not limited to, a fixed bed reactor or a glass reactor tube.

A6. the process according to clause A1, wherein said adsorbent is selected from activated clay, NaY molecular sieve, X-type molecular sieve, activated carbon, preferably activated clay, and NaY molecular sieve.

A7. The process according to clause A1, wherein the pretreatment temperature is from room temperature to 60 °C; the pretreatment pressure is from 0.0 to 0.5 MPa; the mass space velocity of the endo-tetrahydrodicyclopentadiene is from 0.1 to 10.0 $h^{-1}$, and preferably from 0.2 to 1$h^{-1}$.

A8. The process according to clause A1, wherein the active metal of the hydrogenation protect agent is selected from one or more of noble metals Pd, Pt, Ru, Rh and non-noble metals Ni, preferably Ni, Pd, Pt; the support is a non-acidic support and is selected from $Al_2O_3$, $SiO_2$, $ZrO_2$, $TiO_2$, $CeO_2$, and activated carbon, preferably $Al_2O_3$ and $SiO_2$, and so on.

A9. The process according to clause A8, wherein based on the total mass of the hydrogenation protect agent, the non-noble metal loading amount is from 1 to 40%, preferably from 5 to 30%, more preferably from 10 to 20%; the noble metal loading amount is from 0.1 to 10%, preferably from 0.2 to 5%, more preferably from 0.3 to 3%.

A10. The process according to clause A1, wherein the isomerization catalyst is a metal-modified molecular sieve catalyst, the modifying metal is selected from one or more of noble metals Pd, Pt, Ru, Rh, and non-noble metals Ni, the molecular sieve is a Y-type molecular sieve selected from HY, USY, REHY, NTY, and SSY, and preferably from HY, USY, and REHY.

A11. The process according to clause A10, wherein based on the total mass of the isomerization catalyst, the non-noble metal loading amount is from 1 to 20%, preferably from 3 to 15%, more preferably from 5 to 10%; the noble metal loading amount is from 0.05 to 3%, preferably from 0.1 to 1.0%, more preferably from 0.2 to 0.5%.

A12. The process according to clause A1, wherein in the fixed bed reactor, the reaction pressure of the system is from 0.1 to 3MPa of hydrogen, preferably from 0.5 to 1.0MPa of hydrogen.

A13. The process according to clause A1, wherein in the fixed bed reactor, the endo-tetrahydrodicyclopentadiene has a mass space velocity of from 0.5 to 5$h^{-1}$, preferably from 0.5 to 2$h^{-1}$; the volume ratio of hydrogen to liquid is from 100 to 1600, preferably from 600 to 1200.

B1. A catalyst for producing adamantane by isomerization of tetrahydrodicyclopentadiene, which comprises a Y-type molecular sieve and a modifying metal, wherein the producing process of the catalyst comprises: modifying the Y-type molecular sieve with an ammonium fluosilicate solution, wherein the mass ratio of the ammonium fluosilicate to the molecular sieve is from 0.1 to 0.3, carrying out a metal impregnation on the treated molecular sieve with a metal precursor, drying, calcining and reducing in a reducing atmosphere.

B2. The catalyst according to clause B1, wherein the Y-type molecular sieve is a hydrogen-type molecular sieve, preferably one or more of HY, HUSY and REHY.

B3. The catalyst according to clause B1, wherein the Y-type molecular sieve contains less than 0.5%, preferably less than 0.2% by mass of $Na_2O$.

B4. The catalyst according to clause B1, wherein the Y-type molecular sieve is modified with an ammonium fluorosilicate solution, and the modification conditions include: mixing the Y-type molecular sieve with water, uniformly stirring to form slurry, and adding ammonium fluosilicate solution into the slurry of the Y-type molecular sieve according to the requirement that the mass ratio of the ammonium fluosilicate to the molecular sieve is from 0.1 to 0.3.

B5. The catalyst according to clause B1, wherein the modification treatment conditions comprise: a treatment temperature of from 30 to 100 °C, preferably from 50 to 80°C, a treatment pressure of from 0.5 to 5 hr, preferably from 0.5 to 3 hr, and filtering and drying after the treatment.

B6. The catalyst according to clause B1, wherein the metal is one or more selected from Pt, Pd, Au, Ru, Rh, and Ni, and the metal precursor is a salt of the metal.

B7. The catalyst according to clause B1, wherein, when the metal is Pt, Pd, Au, Ru, or Rh, the metal loading amount is from 0.05 to 5.0%, preferably from 0.2 to 0.5%, and when the metal is Ni, the metal loading amount is from 1.0 to 10%, preferably from 3 to 6%.

B8. The catalyst according to clause B1, wherein the concentration of the ammonium fluorosilicate solution is from 0.02 to 2mol/L, preferably from 0.05 to 0.3 mol/L.

B9. The catalyst according to clauses B1 and B4, wherein the mass ratio of the Y-type molecular sieve to water is from 1: 1 to 1:30, preferably from 1:5 to 1:15.

B10. A process for producing adamantane, comprising: mixing the tetrahydrodicyclopentadiene and a solvent in a reactor, and reacting in the presence of hydrogen and the catalyst of one of the clauses B1-9, the reaction temperature is from 181 to 300 °C, preferably from 200 to 260 °C; and the pressure (gauge pressure) of the reaction hydrogen is from 0.1 to 3.0 MPa, preferably from 0.5 to 1.0 MPa.

B11. The process according to clause B10, wherein the endo-tetrahydrodicyclopentadiene has a mass space velocity of from 0.2 to $5h^{-1}$, preferably from 0.5 to $2h^{-1}$; the volume ratio of hydrogen to liquid is from 100 to 3000, preferably from 600 to 1200.

B12. The process according to clause B10, wherein the tetrahydrodicyclopentadiene is mixed with a solvent before the reaction, and the mass concentration of the tetrahydrodicyclopentadiene in the mixed solution is from 10 to 80 wt.%, preferably from 30 to 60 wt.%.

B13. The process according to clause B10, wherein the solvent is a hydrocarbon or halogenated hydrocarbon solvent with a boiling point of from 40 to 300 °C, wherein hydrocarbon or halogenated hydrocarbon solvent is preferably a C6-C10 hydrocarbon, more preferably cyclohexane, methylcyclohexane, exo-tetrahydrodicyclopentadiene or combinations thereof.

B14. The process according to clause B10, wherein the reactor can be a fixed bed reactor, a moving bed reactor, a fluidized bed, a slurry bed, preferably a fixed bed reactor.

Examples

The present application is further described below in conjunction with examples, but the present application is not limited thereby.

Example I Series

The following series of examples I tests the effect of different operating conditions on the process of producing adamantane of the first aspect of this application in a fixed bed reactor comprising a hydrogenation section (i.e. the first reaction zone) and an isomerization section (i.e. the second reaction zone) in which:

endo-tetrahydrodicyclopentadiene is available from Beijing InnoChem Science & Technology Co., Ltd;
quartz sand is filled between the hydrogenation zone and the isomerization zone as an inert material.

$$\text{Conversion rate} = \frac{\text{Endo} - \text{THDCPD initial molar amount} - \text{Endo} - \text{THDCPD residure molar amount}}{\text{Endo} - \text{THDCPD initial molar amount}} \times 100\%$$

$$\text{Selectivity} = \frac{\text{molar amount of product produced}}{\text{molar amount of Endo} - \text{THDCPD converted}} \times 100\%$$

Examples I-1 to I-7 and comparative examples I-1 to I-2 (Influence of the hydrogeneration protective agent)

A methylcyclohexane solution containing 50wt.% of endo-tetrahydrodicyclopentadiene is used as a raw material. The raw material is mixed uniformly in advance and then sequentially passes through a hydrogenation section and an isomerization section.

In a fixed bed reactor, 0.3wt.% of Pt/HY is used as an isomerization catalyst, and the reaction temperature of an isomerization section is 220 °C. The composition of a hydrogenation protective agent and the temperature of a hydrogenation section are shown in Table I-1. The reaction pressure is 0.5MPa of hydrogen. The mass space velocity of endo-tetrahydrodicyclopentadiene is 1 h$^{-1}$. The volume ratio of hydrogen to liquid is 1000. After 20 h of reaction, sampling analysis is carried out to investigate the influence of the hydrogenation protective agent on the reacting performance. The results are shown in Table I-1.

TABLE I-1. test results for examples I-1 to I-7 and comparative examples I-1 to I-2

| Example No. | Hydrogenation protective agent | Hydrogenation section Temperature (°C) | Endo-THDCPD Conversion rate (%) | Exo-THDCPD Selectivity (%) | Adamantane selectivity (%) | Ring-opening by-product selectivity (%) |
|---|---|---|---|---|---|---|
| Example I-1 | 20wt.%Ni/SiO$_2$ | 150 | 98 | 68.4 | 15.0 | 16.6 |
| Example I-2 | 20wt.%Ni/SiO$_2$ | 200 | 99 | 67.7 | 15.2 | 17.1 |
| Example I-3 | 20wt.%Ni/SiO$_2$ | 250 | 99 | 63.6 | 15.0 | 21.4 |
| Example I-4 | 10wt.%Ni/SiO$_2$ | 200 | 98 | 68.4 | 14.0 | 17.6 |
| Example I-5 | 20wt.%Ni/Al$_2$O$_3$ | 200 | 99 | 67.5 | 15.1 | 17.4 |
| Example I-6 | 0.3wt.%Pd/SiO$_2$ | 200 | 99 | 67.7 | 15.0 | 17.3 |
| Example I-7 | 3wt.%Pd/SiO$_2$ | 200 | 99 | 66.9 | 15.5 | 17.6 |
| Comparative example I-1 | 20wt.%Ni/Hβ | 200 | 88 | 68.8 | 13.1 | 18.1 |
| Comparative example I-2 | none | none | 81 | 74.5 | 12.0 | 13.5 |
| *The ring-opening by-products mainly refer to C10 hydrocarbons such as decalin and are calculated based on the molar amount of the C10 component (the same applies below). | | | | | | |

As can be seen from the reaction results of Table I-1, the process of the present application can significantly improve the reaction conversion rate and adamantane selectivity by employing a hydrogenation protective agent.

Examples I-8 to I-9 and comparative examples I-3 to I-4 (Influence of pretreatment process)

In a pretreatment reactor, a methylcyclohexane solution containing 50wt.% of endo-tetrahydrodicyclopentadiene is used as a raw material and flows through a pretreatment reactor filled with different adsorbents at normal temperature and normal pressure, wherein the mass space velocity of the endo-tetrahydrodicyclopentadiene is 0.5h$^{-1}$. Then it is pumped into a fixed bed reactor after pretreatment.

In a fixed bed reactor, methylcyclohexane solutions containing 50wt.% of endo-tetrahydrodicyclopentadiene after different pretreatment processes are used as a raw material; 20wt.%Ni/SiO$_2$ is used as a hydrogenation protective agent, wherein the reaction temperature of a hydrogenation section is 200 °C; 0.3wt.%Pt/HY is used as an isomerization catalyst, the reaction temperature of an isomerization section is 220 °C, the reaction pressure of the entire fixed bed is 0.5MPa of hydrogen, the mass space velocity of the endo-tetrahydrodicyclopentadiene is 1 h$^{-1}$, the volume ratio of hydrogen to liquid is 1000. After 10 h and 50 h of reaction, sampling and analysis are carried out to investigate the influence of the pretreatment processes on the reaction result. The results are shown in Table I-2.

TABLE I-2 test results for examples I-8 to I-9 and comparative examples I-3 to I-4

| Example No. | Pretreatment process | Reaction time | Endo-THDCPD Conversion rate(%) | Exo-THDCPD Selectivity (%) | Adamantane selectivity (%) | Ring-opening by-product selectivity (%) |
|---|---|---|---|---|---|---|
| Comparative example I-3 | Without pretreatment | 10h | 99 | 67.5 | 15.4 | 17.1 |
| | | 50 h | 50 | 83.1 | 6.5 | 10.4 |
| Example I-8 | With NaY pretreatment | 10h | 99 | 67.1 | 15.6 | 17.3 |
| | | 50 h | 99 | 67.2 | 15.6 | 17.2 |
| Example I-9 | With activated clay pretreatment | 10h | 99 | 67.3 | 15.6 | 17.1 |
| | | 50 h | 97 | 68.0 | 15.5 | 16.5 |
| Comparative example I-4 | With HY Pretreatment | 10h | 99 | 67.3 | 15.5 | 17.2 |
| | | 50 h | 52 | 80.8 | 8.4 | 10.8 |

As can be seen from the reaction results of Table I-2, the process of the present application can significantly improve the stability of the reaction system by employing a pretreatment step.

Examples I-10 to I-14 and Comparative examples I-5 to I-6 (Influence of isomerization catalyst)

In the pretreatment reactor, a methylcyclohexane solution containing 50wt% of endo-tetrahydrodicyclopentadiene is used as a raw material. Impurities are removed through NaY pretreatment after the raw material is mixed uniformly in advance.

In a fixed bed reactor, 20 wt% $Ni/SiO_2$ is used as a hydrogenation protection catalyst. The reaction temperature of the hydrogenation protective agent is 200 °C. The temperature of an isomerization section is 220 °C. The compositions of the isomerization catalysts are shown in Table I-3. The reaction pressure is 0.5MPa of hydrogen. The mass space velocity of the endo- tetrahydrodicyclopentadiene is 1 h $^{-1}$. The volume ratio of hydrogen to liquid is 1000. After 50 h of reaction, sampling, and analysis are carried out to investigate the influence of the composition of the isomerization catalyst on the reacting performance. The results are shown in Table I-3.

TABLE I-3 test results for examples I-10 to I-14 and comparative examples I-5 to I-6

| Example No. | Isomerization catalyst | Endo-THDCPD Conversion Rate (%) | Exo-THDCPD Selectivity (%) | Adamantane selectivity (%) | Ring-opening by-product selectivity (%) |
|---|---|---|---|---|---|
| Example I-10 | 0.3wt.%Pt/HY | 99 | 67.2 | 15.6 | 17.2 |
| Example I-11 | 0.1wt.%Pt/HY | 98 | 68.0 | 15.0 | 17.0 |
| Example I-12 | 5wt.%Ni/HY | 97 | 76.1 | 13.6 | 10.3 |
| Example I-13 | 10wt.%Ni/HY | 96 | 72.9 | 14.0 | 13.1 |
| Example I-14 | 0.3wt.%Pt/REHY | 94 | 71.0 | 14.4 | 14.6 |
| Comparative example I-5 | 0.3wt.%Pt/Hβ | 42 | 89 | 2.1 | 8.9 |
| Comparative example I-6 | HY | 31 | 92.1 | 2.2 | 5.7 |

Examples I-15 to I-17 and Comparative example I-7 (Influence of isomerization reaction temperature)

A methylcyclohexane solution containing 50 wt.% of endo-tetrahydrodicyclopentadiene is used as a raw material. Impurities are removed through NaY pretreatment after the raw material is uniformly mixed in advance. 20wt% $Ni/SiO_2$ is used as a hydrogenation protective agent. The temperature of a hydrogenation section is 200 °C. 0.3wt% of Pt/HY is used as an isomerization catalyst. The reaction pressure of the entire fixed bed is 0.5MPa of hydrogen.

The mass space velocity of endo-tetrahydrodicyclopentadiene is 1 h$^{-1}$. The volume ratio of hydrogen to liquid is 1000. After 10 h of reaction, sampling and analysis are carried out to investigate the influence of the isomerization reaction temperature. The results are shown in Table 4.

TABLE I-4 test results for examples I-15 to I-17 and comparative example I-7

| Example No. | Reaction temperature in the isomerization section (°C) | Endo-THDCPD Conversion rate (%) | Exo-THDCPD Selectivity (%) | Adamantane selectivity (%) | Ring-opening by-product selectivity (%) |
|---|---|---|---|---|---|
| Comparative example I-7 | 180 | 96 | 80.7 | 8.6 | 10.7 |
| Example I-15 | 200 | 98 | 71.4 | 13.3 | 15.3 |
| Example I-16 | 220 | 99 | 67.1 | 15.6 | 17.3 |
| Example I-17 | 240 | 99 | 56.6 | 15.8 | 27.6 |

Examples I-18 to I-21 (Influence of reaction pressure)

A methylcyclohexane solution containing 50 wt% of endo-tetrahydrodicyclopentadiene is used as a raw material. Impurities are removed through NaY pretreatment after the raw material is uniformly mixed in advance. 20wt% Ni/SiO$_2$ is taken as a hydrogenation protective agent. The temperature of a hydrogenation section is 200 °C. 0.3wt% of Pt/HY is used as an isomerization catalyst. The reaction temperature of an isomerization section is 220 °C. The mass space velocity of endo-tetrahydrodicyclopentadiene is 1h$^{-1}$. The volume ratio of hydrogen to liquid is 1000. After 10 h of reaction, sampling and analysis are carried out to investigate the influence of reaction pressure of the system at hydrogen atmosphere. The results are shown in Table I-5.

TABLE I-5 test results for examples I-18 to I-21

| Example No. | Reaction pressure (MPa) | Endo-THDCPD Conversion rate (%) | Exo-THDCPD Selectivity (%) | Adamantane selectivity (%) | Ring-opening by-product selectivity (%) |
|---|---|---|---|---|---|
| Example I-18 | 0.2 | 92 | 76.6 | 12.9 | 10.5 |
| Example I-19 | 0.5 | 99 | 67.1 | 15.6 | 17.3 |
| Example I-20 | 1.0 | 99 | 64.0 | 13.7 | 22.3 |
| Example I-21 | 2.0 | 99 | 57.1 | 10.9 | 32.0 |

Examples I-22 to I-25 (Influence of the Mass space velocity)

A methylcyclohexane solution containing 50 wt% of endo-tetrahydrodicyclopentadiene is used as a raw material. Impurities are removed through NaY pretreatment after the raw material is uniformly mixed in advance. 20wt% Ni/SiO$_2$ is taken as a hydrogenation protective agent. The temperature of a hydrogenation section is 200 °C. 0.3wt% Pt/HY is used as an isomerization catalyst. The reaction temperature of an isomerization section is 220 °C. The reaction pressure of the entire fixed bed is 0.5MPa of hydrogen. The volume ratio of hydrogen to liquid is 1000. After 10 h of reaction, sampling and analysis are carried out to investigate the influence of the mass space velocity of endo-tetrahydro dicyclopentadiene. The results are shown in I-Table 6.

TABLE I-6 test results for examples I-22 to I-25

| Example No. | Mass space velocity (h$^{-1}$) | Endo-THDCPD Conversion rate (%) | Exo-THDCPD Selectivity (%) | Adamantane selectivity (%) | Ring-opening by-product selectivity (%) |
|---|---|---|---|---|---|
| Example I-22 | 0.5 | 99 | 59.5 | 15.7 | 24.8 |
| Example I-23 | 1.0 | 99 | 67.1 | 15.6 | 17.3 |
| Example I-24 | 2.0 | 98 | 73.3 | 12.8 | 13.9 |

(continued)

| Example No. | Mass space velocity (h$^{-1}$) | Endo-THDCPD Conversion rate (%) | Exo-THDCPD Selectivity (%) | Adamantane selectivity (%) | Ring-opening by-product selectivity (%) |
|---|---|---|---|---|---|
| Example I-25 | 3.0 | 94 | 79.9 | 9.3 | 10.8 |

Examples I-26 to I-28 (Influence of the volume ratio of hydrogen to liquid)

A methylcyclohexane solution containing 50wt% of endo-tetrahydrodicyclopentadiene is used as a raw material. Impurities are removed through NaY pretreatment after the raw material is uniformly mixed in advance. 20wt% Ni/SiO$_2$ is used as a hydrogenation protective agent. The temperature of a hydrogenation section is 200 °C. 0.3wt% Pt/HY is used as an isomerization catalyst. The reaction temperature of an isomerization section is 220 °C. The reaction pressure of the entire fixed bed is 0.5MPa of hydrogen. The mass space velocity of the endo-tetrahydrodicyclopentadiene is 1h$^{-1}$. After 10 h of reaction, sampling and analysis are carried out to investigate the influence of the volume ratio of hydrogen to liquid. The results are shown in Table I-7.

TABLE I-7 test results for examples I-26 to I-28

| Example No. | Volume ratio of hydrogen to liquid | Endo-THDCPD Conversion rate (%) | Exo-THDCPD Selectivity (%) | Adamantane selectivity (%) | Ring-opening by-product selectivity (%) |
|---|---|---|---|---|---|
| Example I-26 | 500 | 98 | 68.1 | 15.3 | 16.6 |
| Example I-27 | 1000 | 99 | 67.1 | 15.6 | 17.3 |
| Example I-28 | 1500 | 99 | 66.9 | 15.7 | 17.4 |

Examples I-29 to I-32 and comparative example I-8 (Influence of reaction solvent and raw material mass concentration)

Endo-tetrahydro-dicyclopentadiene solutions with different mass concentrations are used as raw materials. Impurities are removed through NaY pretreatment after the raw materials are mixed uniformly in advance. 20wt% Ni/SiO$_2$ is used as a hydrogenation protective agent. The temperature of a hydrogenation section is 200 °C. 0.3wt% Pt/HY is used as an isomerization catalyst. The reaction temperature of an isomerization section is 220 °C. The reaction pressure of the entire fixed bed is 0.5MPa of hydrogen. The mass space velocity of the endo-tetrahydrodicyclopentadiene is 1 h$^{-1}$. The volume ratio of hydrogen to liquid is 1000. After 10 h of reaction, sampling and analysis are carried out to investigate the influences of the different reaction solvents and the mass concentration of raw materials. The results are shown in Table I-8.

TABLE I-8 test results for examples I-29 to I-32 and comparative example I-8

| Example No. | Reaction solvent | Mass concent ration (wt. %) | Endo-THDCPD Conversion rate (%) | Exo-THDCPD Selectivit y (%) | Adamant ane selectivit y (%) | Ring-opening by-product selectivity (%) |
|---|---|---|---|---|---|---|
| Example I-29 | Methyl cyclohexane | 50 | 99 | 67.1 | 15.6 | 17.3 |
| Example I-30 | Exo-tetrahy-drodicy clo-pentadiene | 50 | 99 | 64.2 | 16.3 | 19.5 |
| Example I-31 | Methyl cyclohexane | 80 | 99 | 63.8 | 15.8 | 20.4 |
| Example I-32 | Methyl cyclohexane | 10 | 99 | 74.2 | 13.9 | 11.9 |

(continued)

| Example No. | Reaction solvent | Mass concent ration (wt. %) | Endo-THDCPD Conversion rate (%) | Exo-THDCPD Selectivit y (%) | Adamant ane selectivit y (%) | Ring-opening by-product selectivity (%) |
|---|---|---|---|---|---|---|
| Comparative example I-8 | Isopropyl alcohol | 50 | 11 | 16.3 | 0.9 | 82.8 |

Example I-33 (System stability test)

The operation period of the example I-1 (with NaY pretreatment) is further prolonged to 500 h. The results show that the conversion rate of endo-THDCPD is 99%. The selectivity of exo-THDCPD is 71.2%. The selectivity of adamantane is 14.9%. The selectivity of ring-opening by-products is 13.9%. These results indicate that the system has a better stability, effectively solves the problem of catalyst coking, and has industrial application prospect.

Example II Series

The following series of examples II tests the effect of different isomerization operating conditions on the process for the production of adamantanes of the present application in a fixed bed reactor, in which:
Ammonium fluorosilicate and endo-tetrahydrodicyclopentadiene are available from Beijing InnoChem Science & Technology Co., Ltd.

The specific surface area and pore volume (including mesoporous volume) of the catalyst are measured by a Quantachrome AS-3 and AS-6 type static nitrogen adsorption instrument according to a nitrogen isothermal physical adsorption-desorption process (BET). The test conditions are as follows: the sample is placed in a sample handling system, evacuated to $1.33 \times 10^{-2}$ Pa at 350 °C, and kept at constant temperature and pressure for 15 hours to decontaminate the sample surface. The adsorption amounts and desorption amounts of $N_2$ on the samples under different relative pressures (p/po) are measured at a liquid nitrogen temperature of -196°C to obtain the adsorption-desorption isotherm curves. Then, the isothermal adsorption data with p/po of 0-0.25 in the linear part of the adsorption branch of the $N_2$ adsorption-desorption curve is selected, the specific surface area of the sample is calculated using the BET formula, and the adsorption amount below the specific pressure p/po $\approx$ 0.98 is selected as the pore volume information of the sample.

The total acid content and strong acid content of the catalyst are determined by pyridine adsorption infrared spectroscopy (Py-IR) using an American BIO-RAD FTS3000 Fourier transform infrared spectrometer. Testing conditions: vacuum is applied at 350 °C to $1 \times 10^{-3}$ Pa, wave number range 1400-1700 cm$^{-1}$. Testing process: the pressed sample is placed in an infrared in-situ cell and sealed. It is first evacuated to $1 \times 10^{-3}$ Pa at 350 °C and maintained for 1 h to completely desorb the impurity gas molecules adsorbed by the sample. Then it is cooled to room temperature. Pyridine is put into the in-situ cell at a pressure of 2.67 Pa. The system is balanced for 30 minutes, and heated to 200 °C, evacuated to $1 \times 10^{-3}$ Pa again. The unadsorbed pyridine molecules are remove. The system is kept for 30 minutes, and cooled to room temperature again. It is scanned in the wave number range of from 1300 to 3900 cm$^{-1}$, and the pyridine adsorption infrared absorption spectrum at 200 °C is recorded, then the in-situ infrared absorption cell is moved to the heat treatment area, heated to 350 °C, evacuated to $1 \times 10^{-3}$ Pa and kept for 30 minutes, cooled to room temperature. The pyridine adsorption infrared spectrum at 350 °C is recorded. Then the information for the total acid content and strong acid content can be obtained.

Examples II-1 to II-2 and comparative example II-1 (Influence of the molecular Sieve $Na_2O$ content)

HY molecular sieves with different $Na_2O$ contents are dried and then impregnated with metal Pt. The metal precursor is tetraammineplatinum chloride with a loading amount of 0.3%. The equivalent volume impregnation process is used and the impregnation lasts for 6 hours at room temperature. During the process, it is stirred intermittently. Then it is dried at 80°C, calcined at 450°C for 3 hours, and reduced with hydrogen at 400°C for 3 hours. The bifunctional catalysts with different $Na_2O$ contents are obtained and their catalytic activity is evaluated (results after 10 hours of reaction). Here, a methylcyclohexane solution containing 50 wt.% of endo-tetrahydrodicyclopentadiene (endo-THDCPD) is used as the raw material in a fixed bed reactor. The reaction temperature is 220°C, the reaction pressure (gauge pressure) is 0.5MPa $H_2$, the mass space velocity of endo-THDCPD is 1 h$^{-1}$, and the volume ratio of hydrogen to liquid is 1000. The performance of the catalyst is investigated (results after 10 hours of reaction). The results are shown in Table II-1.

TABLE II-1 test results for examples II-1 to II-2 and comparative example II-1

| Example No. | $Na_2O$ content (%) | Endo-THDCPD Conversion rate (%) | Adamantane Selectivity (%) | By-products selectivity (%) |
|---|---|---|---|---|
| Example II-1 | 0.07 | 93 | 13.6 | 86.4 |
| Example II-2 | 0.18 | 90 | 12.7 | 87.3 |
| Comparative example II-1 | 0.62 | 67 | 8.0 | 92.0 |

Examples II-3 to II-5 and comparative examples II-2 to II-3 (Influence of ammonium fluorosilicate treatment)

The HY molecular sieve ($Na_2O$ content of 0.07%) is dried, then the HY molecular sieve and water are mixed and stirred uniformly to form slurry. Then the slurry is treated with ammonium fluorosilicate. According to the requirement that the mass ratio of ammonium fluorosilicate to HY molecular sieve is 0, 0.1, 0.2, 0.3, and 0.4, 0.1 mol/L ammonium fluorosilicate solution is slowly added to the HY molecular sieve slurry in dropwise, followed by standing at 60 °C for 1 hour. Finally, HY molecular sieves modified with different amounts of ammonium fluorosilicate are obtained after washing and drying. The specific surface area, pore volume, mesoporous volume, acid content, etc. of the catalyst are further characterized and analyzed. At the same time, the metal loading and catalyst activity evaluation (results after 10 hours of reaction) are carried out according to the process described in Example II-1. The results are shown in Table II-2.

TABLE II-2 test results for examples II-3 to II-5 and comparative examples II-2 to II-3

| Example No. | Ammonium fluorosilicate: HY (mass ratio) | Specific surface area (m²/g) | Pore volume (cm³/g) | Mesopore volume (cm³/g) | Total acid amount/(μmo l/g) | | Amount of strong acid/ (μmol/g) | | Endo-THDCPD Conversion rate (%) | Adamantane Selectivity (%) | By-products Selectivity (%) |
|---|---|---|---|---|---|---|---|---|---|---|---|
| | | | | | B acid | L acid | B acid | L acid | | | |
| Comparative example II-2 | 0 | 699 | 0.376 | 0.074 | 378 | 44 | 367 | 27 | 93 | 13.6 | 86.4 |
| Example II-3 | 0.1 | 737 | 0.396 | 0.073 | 569 | 46 | 527 | 32 | 99 | 15.6 | 84.4 |
| Example II-4 | 0.2 | 793 | 0.426 | 0.079 | 712 | 72 | 645 | 65 | 98 | 15.5 | 84.5 |
| Example II-5 | 0.3 | 751 | 0.422 | 0.076 | 446 | 52 | 420 | 45 | 94 | 15.0 | 85.0 |
| Comparative example II-3 | 0.4 | 720 | 0.386 | 0.069 | 137 | 21 | 124 | 18 | 83 | 10.1 | 89.9 |

Examples II-6 to II-12 and comparative example II-4 (Influences of ammonium fluorosilicate concentration and treatment temperature, time)

The HY molecular sieve ($Na_2O$ content of 0.07%) is dried, and then the HY molecular sieve is mixed with water and stirred uniformly to form a slurry. The ammonium fluorosilicate treatment is carried out according to the steps and proportions of Example II-3, but the concentration of the ammonium fluorosilicate solution is adjusted to 0.05 mol/L or 0.3 mol/L. In addition, the treatment temperature of the ammonium fluorosilicate is adjusted to 50°C or 80°C, and the treatment time is adjusted to 0.5h or 3h. The metal loading and catalyst activity evaluation are carried out according to the process described in Example II-1 (results after 10h of reaction). The results are shown in Table II-3.

TABLE II-3 test results for examples II-6 to II-12 and comparative example II-4

| Examples | Ammonium fluorosilicate concentration/ (mol/L) | Treatment temperature/ °C | Treatment time/ h | Endo-THDCPD Conversion rate (%) | Adamantane Selectivity (%) | By-products selectivity (%) |
|---|---|---|---|---|---|---|
| Comparative example II-4 | 0 | 60 | 1 | 93 | 13.6 | 86.4 |
| Example II-6 | 0.05 | 60 | 1 | 95 | 14.4 | 85.6 |
| Example II-7 | 0.1 | 60 | 1 | 99 | 15.6 | 84.4 |
| Example II-8 | 0.3 | 60 | 1 | 97 | 15.0 | 85.0 |
| Example II-9 | 0.1 | 50 | 1 | 98 | 15.1 | 84.9 |
| ExampleII-10 | 0.1 | 80 | 1 | 96 | 13.7 | 86.3 |
| Example II-11 | 0.1 | 60 | 0.5 | 95 | 14.0 | 86.0 |
| Example 11-12 | 0.1 | 60 | 3 | 94 | 13.8 | 86.2 |

Examples II-13 to II-16 and comparative examples II-5 to II-7 (Influence of the loaded metal)

The HY molecular sieve ($Na_2O$ content of 0.07%) is dried and then treated with ammonium fluorosilicate. According to the steps of Example II-3, the ammonium fluorosilicate solution is slowly added to the HY molecular sieve slurry in dropwise according to the requirement that the mass ratio of ammonium fluorosilicate to HY molecular sieve is 0.1. Then it is allowed to stand for 1 hour at 60°C, and finally washed and dried to obtain the HY molecular sieve modified with ammonium fluorosilicate. Subsequently, the raw material is pretreated by adsorption using NaY according to the process in Example I-1. Then the metal loading and catalyst activity evaluation (results after 50 hours of reaction) are carried out according to the process described in Example II-1, but the type and loading amount of the loaded metal are adjusted, and the results are shown in Table II-4.

TABLE II-4 Test results for Examples II-13 to II-16 and Comparative examples II-5 to II-7

| Examples | Type and amount of loaded metal | Endo-THDCPD Conversion rate (%) | Adamantane Selectivity (%) | By-products selectivity (%) |
|---|---|---|---|---|
| Comparative example II-5 | none | 47 | 4.8 | 95.2 |
| Example II-13 | 0.3%Pt | 99 | 15.6 | 84.4 |
| Example II-14 | 0.1 %Pt | 86 | 11.6 | 88.4 |
| Example II-15 | 0.3%Pd | 99 | 15.8 | 84.2 |
| Example II-16 | 5%Ni | 92 | 13.3 | 86.7 |
| Comparative example II-6 | 5%Cu | 42 | 3.9 | 96.1 |
| Comparative example II-7 | 15%Ni | 76 | 7.6 | 92.4 |

Examples II-17 to II-20 and comparative example II-8 (Influence of reaction temperature)
The HY molecular sieve ($Na_2O$ content of 0.07%) is dried and then treated with ammonium fluorosilicate.

According to the steps of Example II-3, the ammonium fluorosilicate solution is slowly added to the HY molecular sieve slurry in dropwise, according to the requirement that the mass ratio of ammonium fluorosilicate to HY molecular sieve is 0.1. Then it is allowed to stand at 60°C for 1h, and finally washed and dried to obtain the HY molecular sieve modified with ammonium fluorosilicate. Subsequently, the metal loading and catalyst activity evaluation (results after 10h of reaction) are carried out according to the process described in Example II-1, but the reaction temperatures are adjusted to 180°C, 260°C, and 300°C, respectively. The results are shown in Table II-5.

TABLE II-5 test results for examples II-17 to II-20 and comparative example II-8

| Examples | Reaction temperature | Endo-THDCPD Conversion rate (%) | Adamantane Selectivity (%) | By-products selectivity (%) |
|---|---|---|---|---|
| Comparative example II-8 | 180 | 99 | 6.9 | 93.1 |
| Example II-17 | 200 | 99 | 13.1 | 86.9 |
| Example II-18 | 220 | 99 | 15.6 | 84.4 |
| Example II-19 | 260 | 99 | 17.4 | 82.6 |
| Example II-20 | 300 | 99 | 12.5 | 87.5 |

Examples II-21 to II-23 and comparative example II-9 (Influence of reaction pressure)

The HY molecular sieve ($Na_2O$ content of 0.07%) is dried and then treated with ammonium fluorosilicate. According to the steps of Example II-3, the ammonium fluorosilicate solution is slowly added to the HY molecular sieve slurry in dropwise, according to the requirement that the mass ratio of ammonium fluorosilicate to HY molecular sieve is 0.1, and then it is allowed to stand for 1 hour at 60°C, and finally washed and dried to obtain the HY molecular sieve modified with ammonium fluorosilicate. Subsequently, the metal loading and catalyst activity evaluation (results after 10 hours of reaction) are carried out according to the process described in Example II-1, but the reaction hydrogen pressure (gauge pressure) is adjusted to 0MPa, 0.5MPa, 1.0MPa, 2.0MPa. The results are shown in Table II-6.

TABLE II-6 test results for examples II-21 to II-23 and comparative example II-9

| Examples | Reaction pressure/MPa | Endo-THDCPD Conversion rate (%) | Adamantane Selectivity (%) | By-products selectivity (%) |
|---|---|---|---|---|
| Comparative example II-9 | 0.0 | 90 | 12.2 | 87.8 |
| Example II-21 | 0.5 | 99 | 15.6 | 84.4 |
| Example II-22 | 1.0 | 99 | 15.1 | 84.9 |
| Example II-23 | 2.0 | 99 | 11.8 | 88.2 |

Examples II-24 to II-27 (Influence of Mass space velocity)

The HY molecular sieve ($Na_2O$ content of 0.07%) is dried and then treated with ammonium fluorosilicate. According to the steps of Example II-3, the ammonium fluorosilicate solution is slowly added to the HY molecular sieve slurry in dropwise according to the requirement that the mass ratio of ammonium fluorosilicate to HY molecular sieve is 0.1, and then it is allowed to stand for 1 hour at 60°C, and finally washed and dried to obtain the HY molecular sieve modified with ammonium fluorosilicate. Subsequently, the metal loading and catalyst activity evaluation (results after 10 hours of reaction) are carried out according to the process described in Example II-1, but the mass space velocity of endo-tetrahydrodicyclopentadiene is adjusted to 0.5h$^{-1}$, 1.0h$^{-1}$, 2.0h$^{-1}$, 4.0h$^{-1}$. The results are shown in Table II-7.

TABLE II-7 test results for examples II-24 to II-27

| Examples | Mass space velocity/ h$^{-1}$ | Endo-THDCPD Conversion rate (%) | Adamantane Selectivity (%) | By-products selectivity (%) |
|---|---|---|---|---|
| Example II-24 | 0.5 | 99 | 17.7 | 82.3 |
| Example II-25 | 1.0 | 99 | 15.6 | 84.4 |
| Example II-26 | 2.0 | 99 | 14.3 | 85.7 |
| Example II-27 | 4.0 | 94 | 8.5 | 91.5 |

Examples II-28 to II-30 and comparative example II-10 (influence of reaction solvent)

The HY molecular sieve (Na$_2$O content of 0.07%) is dried and then treated with ammonium fluorosilicate. According to the steps of Example II-3, the ammonium fluorosilicate solution is slowly added to the HY molecular sieve slurry in dropwise according to the requirement that the mass ratio of ammonium fluorosilicate to HY molecular sieve is 0.1, and then it is allowed to stand at 60°C for 1h, and finally washed and dried to obtain the HY molecular sieve modified with ammonium fluorosilicate. Subsequently, the metal loading and catalyst activity evaluation (results after 10h of reaction) are carried out according to the process described in Example II-1, but the reaction solvent is replaced with exo-tetrahydrodicyclopentadiene, dichloromethane, and isopropanol. The results are shown in Table II-8.

TABLE II-8 test results for examples II-28 to II-30 and comparative example II-10

| Examples | Reaction solvent | Endo-THDCPD Conversion rate (%) | Adamantane Selectivity (%) | By-products selectivity (%) |
|---|---|---|---|---|
| Example II-28 | Methyl cyclohexane | 99 | 15.6 | 84.4 |
| Example II-29 | Exo-tetrahydrodicyclopentadiene | 99 | 16.4 | 83.6 |
| Example II-30 | Methylene dichloride | 99 | 15.9 | 84.1 |
| Comparative example 11-10 | Isopropyl alcohol | 11 | 2.7 | 97.3 |

The preferred embodiments of the present application are described in detail above, but the present application is not limited to the specific details in the above embodiments. Within the technical concept of the present application, the technical solution of the present application can be subjected to a variety of simple modifications, and these simple modifications all belong to the protection scope of the present application.

It should also be noted that the various specific technical features described in the above specific embodiments can be combined in any suitable manner if there is no contradiction. In order to avoid unnecessary repetition, the present application will not further describe various possible combinations.

In addition, the various different implementations of the present application can also be arbitrarily combined, as long as they do not violate the idea of the present application, they should also be regarded as the content invented by the present application.

## Claims

1.   A continuous process for producing adamantine, comprising the steps of:

1) providing a liquid feed stream comprising endo-tetrahydrodicyclopentadiene;
2) passing the liquid feed stream through a first reaction zone filled with a hydrogenation protective agent and a second reaction zone filled with an isomerization catalyst sequentially, to carry out hydroisomerization reaction, thereby obtaining adamantane,
wherein the reaction temperature in the first reaction zone is from 120 to 300 °C, preferably from 151 to 250 °C, the reaction temperature in the second reaction zone is from 181 to 300 °C, preferably from 200 to 260 °C, the hydrogenation protective agent is a loaded metal hydrogenation catalyst, and the isomerization catalyst is a metal

modified molecular sieve catalyst.

2. The process according to claim 1, wherein the liquid feed stream comprises endo-tetrahydrodicyclopentadiene and a reaction solvent selected from hydrocarbon or halogenated hydrocarbon solvents having a boiling point of 40-300 °C, the hydrocarbon or halogenated hydrocarbon solvents are preferably selected from C6-C10 hydrocarbons, more preferably selected from cyclohexane, methylcyclohexane, exo-tetrahydrodicyclopentadiene, or combinations thereof;

preferably, the concentration by mass of theendo-tetrahydrodicyclopentadiene in the feed stream is from 10 to 80%, more preferably from 30 to 60%.

3. The process according to claim 1 or 2, wherein step 1) further comprises pretreating the liquid feed stream comprising endo-tetrahydrodicyclopentadiene with an adsorbent,

preferably, the adsorbent is selected from activated clay, NaY molecular sieve, X-type molecular sieve, activated carbon, or combinations thereof, preferably selected from activated clay, NaY molecular sieve, or combinations thereof,

more preferably, the conditions of the pretreatment of step 1) include: the temperature of from normal temperature to 60 °C, the pressure of from 0.0 to 0.5 MPa, and the mass space velocity of the endo-tetrahydrodicyclopentadiene of from 0.1 to 10.0 h$^{-1}$, preferably from 0.2 to 1 h$^{-1}$.

4. The process according to any of the preceding claims, wherein the hydrogenation protective agent comprises a support and an active metal supported on the support, wherein the active metal is selected from Pd, Pt, Ru, Rh, Ni, or combinations thereof, preferably from Ni, Pd, Pt, or combinations thereof; the support is a non-acidic support selected from $Al_2O_3$, $SiO_2$, $ZrO_2$, $TiO_2$, $CeO_2$, activated carbon or combinations thereof, preferably from $Al_2O_3$, $SiO_2$, or combinations thereof;

preferably, based on the total mass of the hydrogenation protective agent, the Ni content in the hydrogenation protective agent is from 1 to 40%, preferably from 5 to 30%, and more preferably from 10 to 20%; and/or the total content of Pd, Pt, Ru and Rh is from 0.1 to 10%, preferably from 0.2 to 5%, more preferably from 0.3 to 3%.

5. The process according to any of the preceding claims, wherein the isomerization catalyst comprises a molecular sieve and a modifying metal supported on the molecular sieve, wherein the modifying metal is selected from Pd, Pt, Au, Ru, Rh, Ni, or combinations thereof, the molecular sieve is a Y-type molecular sieve, preferably selected from HY, USY, REHY, or combinations thereof, more preferably selected from HY, HUSY, REHY, or combinations thereof;

preferably, based on the total mass of the isomerization catalyst, the content of Ni in the isomerization catalyst is from 1 to 20%, preferably from 3 to 15%, more preferably from 3 to 10%; and/or the total content of Pd, Pt, Au, Ru and Rh is from 0.05 to 5.0%, preferably from 0.1 to 1.0%, more preferably from 0.2 to 0.5%;

more preferably, the molecular sieve has a $Na_2O$ content of less than 0.5%, preferably less than 0.2%, based on the mass of the molecular sieve.

6. The process according to claim 5, wherein the isomerization catalyst has a specific surface area of from 450 to 900 m$^2$/g, preferably from 600 to 800 m$^2$/g, a pore volume of from 0.25 to 0.5 cm$^3$/g, preferably from 0.35 to 0.45 cm$^3$/g, a mesoporous volume of from 0.02 to 0.10 cm$^3$/g, preferably from 0.07 to 0.09 cm$^3$/g, a strong acid amount of from 150 to 850 $\mu$mol/g, preferably from 370 to 700 $\mu$mol/g.

7. The process according to claim 5 or 6, wherein the isomerization catalyst is prepared by a process comprising the steps of:

I) modifying the Y-type molecular sieve by using an ammonium fluosilicate solution, wherein the mass ratio of the ammonium fluosilicate to the Y-type molecular sieve is from 0.1 to 0.3, and the Y-type molecular sieve is preferably a hydrogen-type molecular sieve, more preferably selected from HY, HUSY, REHY or combinations thereof; and
II) loading the modifying metal on the modified molecular sieve obtained in the step I), calcining, and reducing in a reducing atmosphere to obtain the isomerization catalyst;

preferably, the loading of step II) is achieved by impregnating the modified molecular sieve with a solution of a precursor of the modifying metal, and optionally drying, wherein the precursor is preferably a salt of said modifying metal.

8. The process according to claim 7, wherein the conditions of the modification treatment in step I) comprise: a treatment temperature of from 30 to 100 °C, preferably from 50 to 80 °C, and a treatment time of from 0.5 to 5h, preferably from 0.5 to 3 h;

preferably, the concentration of the ammonium fluosilicate solution is from 0.02 to 2 mol/L, preferably from 0.05 to 0.3 mol/L.

9.  The process according to any one of the preceding claims, wherein the first and second reaction zones are under a hydrogen atmosphere and the reaction pressure is from 0.1 to 3MPa, preferably from 0.5 to 1.0 MPa;

preferably, the mass space velocity of the endo-tetrahydrodicyclopentadiene in the first and second reaction zones is from 0.5 to 5 h$^{-1}$, preferably from 0.5 to 2 h$^{-1}$; the volume ratio of hydrogen to liquid is from 100 to 1600, preferably from 600 to 1200.

10.  The process according to any of the preceding claims, wherein step 2) is carried out with a fixed bed reactor and the first reaction zone is arranged above the second reaction zone, preferably the first reaction zone and the second reaction zone are separated by an inert material.

11.  A process for producing adamantane, comprising contacting a liquid feed stream containing tetrahydrodicyclopentadiene, especially endo-tetrahydrodicyclopentadiene, with an isomerization catalyst under a hydrogen atmosphere to carry out isomerization reaction, wherein the isomerization catalyst is a metal modified molecular sieve catalyst with a specific surface area of from 450 to 900 m$^2$/g, preferably from 600 to 800 m$^2$/g, a pore volume of from 0.25 to 0.5 cm$^3$/g, preferably from 0.35 to 0.45 cm$^3$/g, a mesoporous volume of from 0.02 to 0.10 cm$^3$/g, preferably from 0.07 to 0.09 cm$^3$/g, the strong acid amount of from 150 to 850 $\mu$mol/g, preferably from 370 to 700 $\mu$mol/g, a reaction temperature of from 181 to 300 °C, preferably from 200 to 260 °C; the reaction pressure is from 0.1 to 3.0 MPa, preferably from 0.5 to 1.0MPa,

preferably, in the reaction, the mass space velocity of the endo-tetrahydrodicyclopentadiene is from 0.2 to 5 h$^{-1}$, preferably from 0.5 to 2 h$^{-1}$; the volume ratio of hydrogen to liquid is from 100 to 3000, preferably from 600 to 1200.

12.  The process according to claim 11, wherein the isomerization catalyst comprises a molecular sieve and a modifying metal supported on the molecular sieve, wherein the modifying metal is selected from Pd, Pt, Au, Ru, Rh, Ni, or combinations thereof, the molecular sieve is a Y-type molecular sieve, preferably selected from HY, USY, REHY, or combinations thereof, more preferably selected from HY, HUSY, REHY, or combinations thereof;

preferably, based on the total mass of the isomerization catalyst, the content of Ni in the isomerization catalyst is from 1 to 20%, preferably from 3 to 15%, more preferably from 3 to 10%; and/or the total content of Pd, Pt, Au, Ru and Rh is from 0.05 to 5.0%, preferably from 0.1 to 1.0%, more preferably from 0.2 to 0.5%;

more preferably, the molecular sieve has a Na$_2$O content of less than 0.5%, preferably less than 0.2%, based on the mass of the molecular sieve.

13.  The process according to claim 11 or 12, wherein the isomerization catalyst is prepared by a process comprising the steps of:

I) modifying the Y-type molecular sieve by using an ammonium fluosilicate solution, wherein the mass ratio of the ammonium fluosilicate to the Y-type molecular sieve is from 0.1 to 0.3, and the Y-type molecular sieve is preferably a hydrogen-type molecular sieve, more preferably selected from HY, HUSY, REHY or combinations thereof; and

II) loading modifying metal on the modified molecular sieve obtained in the step I), calcining, and reducing in a reducing atmosphere to obtain the isomerization catalyst;

preferably, the loading of step II) is achieved by impregnating the modified molecular sieve with a solution of a precursor of the modifying metal, and optionally drying, wherein the precursor is preferably a salt of said modifying metal.

14.  The process according to claim 13, wherein the conditions of the modification treatment in step I) comprise: a treatment temperature of from 30 to 100 °C, preferably from 50 to 80 °C, and a treatment time of from 0.5 to 5h, preferably from 0.5 to 3 h;

preferably, the concentration of the ammonium fluosilicate solution is from 0.02 to 2 mol/L, preferably from 0.05 to 0.3 mol/L.

15.  The process according to any one of claims 11 to 14, wherein the feed stream comprises endo-tetrahydrodicyclopentadiene and a reaction solvent selected from hydrocarbon or halogenated hydrocarbon solvents having a boiling point of 40-300 °C, wherein the hydrocarbon or halogenated hydrocarbon solvents are preferably selected from C6-C10 hydrocarbons, more preferably selected from cyclohexane, methylcyclohexane, exo-tetrahydrodicyclopentadiene, or combinations thereof;

preferably, the concentration by mass of the endo-tetrahydrodicyclopentadiene in the feed stream is from 10 to 80%, more preferably from 30 to 60%.

16. The process according to any one of claims 11 to 15, wherein the isomerization reaction is carried out in a reactor selected from a fixed bed, a moving bed, a fluidized bed, a slurry bed, or combinations thereof, preferably in a fixed bed reactor.

17. The process according to any one of claims 11 to 16, further comprising, prior to performing the isomerization reaction, pretreating the liquid feed stream comprising tetrahydrodicyclopentadiene with an adsorbent,

preferably, the adsorbent is selected from activated clay, NaY molecular sieve, X-type molecular sieve, activated carbon, or combinations thereof, more preferably selected from activated clay, NaY molecular sieve, or combinations thereof,

further preferably, the conditions of the pretreatment include: the temperature of from normal temperature to 60 °C, the pressure of from 0.0 to 0.5 MPa, and the mass space velocity of the endo-tetrahydrodicyclopentadiene of from 0.1 to 10.0 $h^{-1}$, preferably from 0.2 to 1 $h^{-1}$.

## INTERNATIONAL SEARCH REPORT

International application No.

**PCT/CN2023/102310**

### A. CLASSIFICATION OF SUBJECT MATTER

C07C 5/29(2006.01)i; C07C 13/615(2006.01)i; B01J 29/12(2006.01)i; B01J 29/14(2006.01)i; C07C 5/29(2006.01)i; C07C 13/615(2006.01)i

According to International Patent Classification (IPC) or to both national classification and IPC

### B. FIELDS SEARCHED

Minimum documentation searched (classification system followed by classification symbols)

IPC: C07C B01J

Documentation searched other than minimum documentation to the extent that such documents are included in the fields searched

Electronic data base consulted during the international search (name of data base and, where practicable, search terms used)

CNABS; DWPI; VEN; ENTXT; ENTXTC; CJFD; CNKI; CNTXT; USTXT; WOTXT; EPTXT; ISI-Web of Science; 万方, WANFANG; 超星读秀, DUXIU: 中国石油化工股份有限公司, 中石化石油化工科学研究院有限公司, 伏朝林, 赵杰, 闫瑞, 贾丹丹, 朱忠朋, 金刚烷, 桥式四氢双环戊二烯, 加氢, 异构体, 分子筛, 催化, 活性炭, Y分子筛, catalyst, ADH, endo-THDCPD, Pd, Pt, Ru, Rh, Ni, Al2O3, SiO2, ZrO2, TiO2, CeO2, HY, USY, REHY, tetrahydrodicyclopentadiene, hydrogenation, isomerization, adamantane

### C. DOCUMENTS CONSIDERED TO BE RELEVANT

| Category* | Citation of document, with indication, where appropriate, of the relevant passages | Relevant to claim No. |
|---|---|---|
| X | CN 1398245 A (IDEMITSU PETROCHEMICAL CO., LTD.) 19 February 2003 (2003-02-19) description, pp. 2 and 3 | 11, 12 |
| X | 郭建维 等 (GUO, Jianwei et al.). "双环戊二烯加氢异构化合成金刚烷 (Non-official translation: Hydroisomerization of Dicyclopentadiene to Synthesize Adamantane)" 催化学报 (Chinese Journal of Catalysis), Vol. vol. 22, No. 3, 31 May 2001 (2001-05-31), pp. 271-274 pp. 271, 273, and 274 | 11, 12 |
| Y | CN 1398245 A (IDEMITSU PETROCHEMICAL CO., LTD.) 19 February 2003 (2003-02-19) description, pp. 2-3 | 13-16 |

☑ Further documents are listed in the continuation of Box C.　　☑ See patent family annex.

| | | |
|---|---|---|
| * | Special categories of cited documents: | |
| "A" | document defining the general state of the art which is not considered to be of particular relevance | "T" later document published after the international filing date or priority date and not in conflict with the application but cited to understand the principle or theory underlying the invention |
| "D" | document cited by the applicant in the international application | "X" document of particular relevance; the claimed invention cannot be |
| "E" | earlier application or patent but published on or after the international filing date | considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" document member of the same patent family |
| "P" | document published prior to the international filing date but later than the priority date claimed | |

| Date of the actual completion of the international search | Date of mailing of the international search report |
|---|---|
| **21 September 2023** | **08 October 2023** |

| Name and mailing address of the ISA/CN | Authorized officer |
|---|---|
| **China National Intellectual Property Administration (ISA/CN) China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/102310**

**C.    DOCUMENTS CONSIDERED TO BE RELEVANT**

| Category* | Citation of document, with indication, where appropriate, of the relevant passages | Relevant to claim No. |
|---|---|---|
| Y | 郭建维 等 (GUO, Jianwei et al.). "双环戊二烯加氢异构化合成金刚烷 (Non-official translation: Hydroisomerization of Dicyclopentadiene to Synthesize Adamantane)" 催化学报 (C鱼inese Journal of Catalysis), Vol. vol. 22, No. 3, 31 May 2001 (2001-05-31), pp. 271-274 pp. 271, 273, and 274 | 13-16 |
| Y | GOLA.A et al. "Effect Of Leaching Agentin the Dealumination of Stabilized Y Zeolites" Microporous and Mesoporous Materials, Vol. 40,, No. 1-3, 30 November 2000 (2000-11-30), pp. 73-83 pp. 76-78, 81, and 82 | 13-16 |
| A | CN 1398245 A (IDEMITSU PETROCHEMICAL CO., LTD.) 19 February 2003 (2003-02-19) description, pp. 2 and 3 | 1-10, 17 |
| A | 郭建维 等 (GUO, Jianwei et al.). "双环戊二烯加氢异构化合成金刚烷 (Non-official translation: Hydroisomerization of Dicyclopentadiene to Synthesize Adamantane)" 催化学报 (C鱼inese Journal of Catalysis), Vol. vol. 22, No. 3, 31 May 2001 (2001-05-31), pp. 271-274 pp. 271, 273, and 274 | 1-10, 17 |
| A | CN 101125791 A (DALIAN UNIVERSITY OF TECHNOLOGY) 20 February 2008 (2008-02-20) entire document | 1-17 |
| A | CN 101229990 A (LUZHOU DAZHOU CHEMICAL CO., LTD.) 30 July 2008 (2008-07-30) entire document | 1-17 |
| A | 高娜 等 (GAO, Na et al.). "负载Pt的分子筛催化剂研究进展 (Advances in Pt-Loading Zeolite Catalysts)" 化工进展 (Chemical Industry and Engineering Progress), Vol. 35, No. 2, 29 February 2016 (2016-02-29), pp. 506-512 p. 509 | 1-17, |

Form PCT/ISA/210 (second sheet) (July 2022)

**INTERNATIONAL SEARCH REPORT**
**Information on patent family members**

International application No.

**PCT/CN2023/102310**

| Patent document cited in search report | | | Publication date (day/month/year) | Patent family member(s) | | | Publication date (day/month/year) |
|---|---|---|---|---|---|---|---|
| CN | 1398245 | A | 19 February 2003 | WO | 0248077 | A1 | 20 June 2002 |
| | | | | EP | 1342708 | A1 | 10 September 2003 |
| | | | | EP | 1342708 | A4 | 31 March 2004 |
| | | | | KR | 20020077430 | A | 11 October 2002 |
| | | | | US | 2003018226 | A1 | 23 January 2003 |
| | | | | JP | 2004051484 | A | 19 February 2004 |
| | | | | CZ | 20022684 | A3 | 13 November 2002 |
| CN | 101125791 | A | 20 February 2008 | None | | | |
| CN | 101229990 | A | 30 July 2008 | CN | 101229990 | B | 29 December 2010 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 1398245 A

**Non-patent literature cited in the description**

- *Synthesis of adamantane on commercially available zeolitic catalysts, Applied Catalysis A: General*, 2000, 127-132

- *Synthesis of adamantane on PW/USY composite catalyst, high-grade chemical engineering report*, 2007, 127-132